# FASCICULE DE BREVET EUROPEEN

(11) **EP 2 477 988 B1**
(45) Date de publication et mention de la délivrance du brevet: **17.07.2013**
(21) Numéro de dépôt: 10770577.4
(22) Date de dépôt: 17.09.2010
(51) Int. Cl.: C07D 471/04, A61K 31/437, A61P 25/00, A61P 25/08, A61P 29/00, A61P 9/10, A61P 35/00

(54) **Dérivés acétyléniques de 5-phényl-pyrazolopyridine, leur préparation et leur application en thérapeutique**
Acetylenderivate des 5-Phenylpyrazolopyridins, Verfahren zu ihrer Herstellung und ihre therapeutische Anwendung
Acetylenic derivatives of 5-phenyl-pyrazolopyridine, process for their preparation and therapeutic use thereof

(30) Priorité: 18.09.2009 FR 0956445
(43) Date de publication de la demande: 25.07.2012
(73) Titulaire: SANOFI, 75008 Paris (FR)
(72) Inventeur: AUGER, Florian, F-75013 Paris (FR); EVEN, Luc, F-75013 Paris (FR)
(74) Mandataire: Le Coupanec, Pascale A.M.P.
(86) Numéro de dépôt international: PCT/FR2010/051931
(87) Numéro de publication internationale: WO 2011/033229

(56) Documents cités:
- EP-A1- 1 389 618
- WO-A1-2004/026871
- WO-A2-01/83479
- SCHWEIZER E E ET AL: "Reactions of azines. 9. Preparation of 4,5-dihydropyrazolo[1,5-a]pyri dines, 6,7-dihydropyrazolo[1,5-a]pyridines, and pyrazolo[1,5-a]pyridines", JOURNAL OF ORGANIC CHEMISTRY, AMERICAN CHEMICAL SOCIETY, EASTON.; US LNKD- DOI:10.1021/JO00383A028, vol. 52, no. 7, 1 avril 1987 (1987-04-01), pages 1319-1324, XP002566492, ISSN: 0022-3263

## Description

La présente invention se rapporte à des dérivés acétyléniques de 5-phénylpyrazolopyridine, à leur préparation et à leur application en thérapeutique dans le traitement ou la prévention de maladies impliquant les récepteurs nucléaires Nurr-1, aussi appelés NR4A2, NOT, TINUR, RNR-1, et HZF3. Des dérivés pyrazolopyridine non acétyléniques à usage thérapeutique sont décrits dans les demandes EP-A-1389618, WO-A-01/83479 et WO-A-2004/026871.

La présente invention a pour objet les composés de formule (I) : 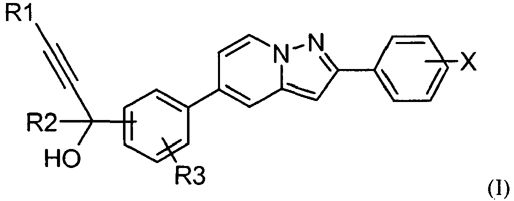 dans laquelle :
R₁ et R2 représentent, indépendamment l'un de l'autre, un atome d'hydrogène ou un groupe (C₁-C₆)alkyle,
R3 représente un ou plusieurs atomes d'hydrogène ou d'halogène,
X représente de 1 à 4 substituants identiques ou différents l'un de l'autre choisis parmi hydrogène, halogène ou (C₁-C₆)alkyle,
à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention, on entend par :
- un groupe (Cₓ-Cₜ) : un groupe comprenant entre x et t atomes de carbone ;
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyle, cyclopropylméthyle etc.

Parmi les composés de formule (I) objets de l'invention, un premier groupe de composés est constitué des composés pour lesquels :
X représente un atome d'halogène ou un groupe (C₁-C₆)alkyle;
R1 et R2 représentent un atome d'hydrogène ou un groupe (C₁-C₆)alkyle ;
R3 représente ou plusieurs atomes d'hydrogène ou d'halogène,
à l'état de base ou de sel d'addition à un acide.

Parmi les composés de formule (I) objets de l'invention, un second groupe de composés est constitué des composés pour lesquels :
X représente un chlore, un fluor ou un groupe méthyle;
R1 et R2 représentent un atome d'hydrogène ou un groupe méthyle ;
R3 représente un atome d'hydrogène ou un groupe difluoro,
à l'état de base ou de sel d'addition à un acide.

Parmi les composés de formule (I) objets de l'invention, on peut notamment citer les composés suivants :
1-{3-[2-(4-Chlorophényl)pyrazolo[1,5-*a*]pyridin-5-yl]phényl}prop-2-yn-1-ol

1-{3-[2-(4-Fluorophényl)pyrazolo[1,5-*a*]pyridin-5-yl]phényl}prop-2-yn-1-ol
1-{3-[2-(4-Fluorophényl)pyrazolo[1,5-*a*]pyridin-5-yl]phényl}but-2-yn-1-ol
2-{3-[2-(4-Fluorophényl)pyrazolo[1,5-*a*]pyridin-5-yl]phényl}but-3-yn-2-ol
1-{2-[2-(4-Fluoro-phényl)pyrazolo[1,5-*a*]pyridin-5-yl]phényl}prop-2-yn-1-ol

1-{2,6-Difluoro-3-[2-(4-fluorophényl)pyrazolo[1,5-*a*]pyridin-5-yl]phényl}prop-2-yn-1-ol
1-{3-[2-(2-Fluorophényl)pyrazolo[1,5-*a*]pyridin-5-yl]phényl}prop-2-yn-1-ol
1-[3-(2-p-Tolylpyrazolo[1,5-*a*]pyridin-5-yl)phényl]prop-2-yn-1-ol
(-)-1-{3-[2-(4-Fluorophényl)pyrazolo[1,5-*a*]pyridin-5-yl]phényl}prop-2-yn-1-ol (+)-1-{3-[2-(4-fluorophényl)pyrazolo[1,5-*a*]pyridin-5-yl]phényl}prop-2-yn-1-ol
(-)-2-{3-[2-(4-Fluorophényl)pyrazolo[1,5-*a*]pyridin-5-yl]phényl}but-3-yn-2-ol
(+)-2-{3-[2-(4-Fluorophényl)pyrazolo[1,5-*a*]pyridin-5-yl]phényl}but-3-yn-2-ol

Conformément à l'invention, on peut préparer les composés de formule générale (I) selon le procédé décrit dans le schéma 1.

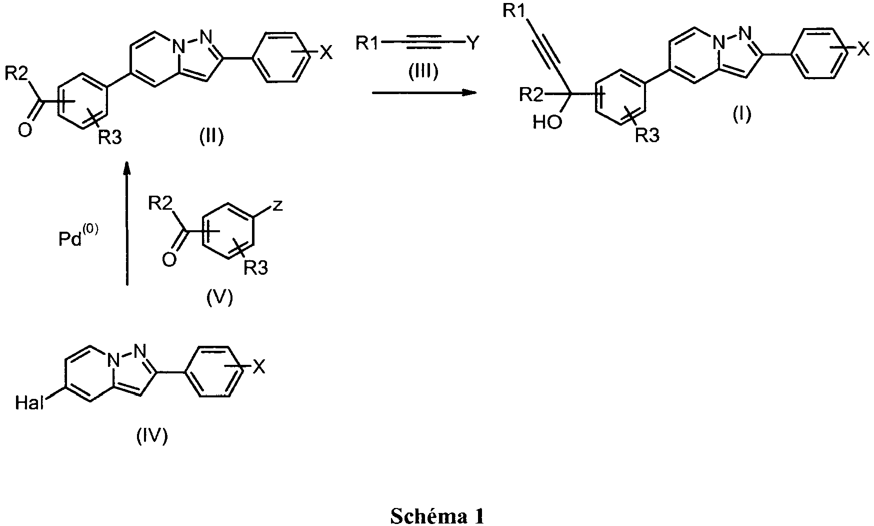

On peut préparer les composés de l'invention suivant le schéma 1 par action d'un dérivé organométallique de formule générale (III) dans laquelle R1 est défini comme précédemment et Y représente un dérivé de métal, par exemple un halogénure de magnésium, sur un dérivé carbonylé de formule générale (II) dans laquelle R2, R3 et X sont définis comme précédemment, pour obtenir les composés de formule générale (I), par exemple selon la méthode décrite par S. Chassaing, M. Kueny-Stotz, G. Isorez et R. Brouillard dans Eur. J. Org. Chem., 2007, 2438.

Les composés de formule générale (II) dans laquelle R2, R3 et X sont définis comme précédemment peuvent être obtenus par une réaction de couplage, catalysée par un métal tel que le palladium, entre une pyrazolopyridine de formule générale (IV), dans laquelle X est défini comme précédemment et Hal représente un atome d'halogène et un dérivé carbonylé de formule générale (V) dans laquelle R2 et R3 sont définis comme précédemment et Z représente un dérivé de bore ou d'étain, par exemple selon la méthode décrite par A. Gueiffier dans Helv. Chim. Acta, 2001, 84, 3610.

Conformément à l'invention, on peut préparer les composés de formule générale (IV), selon le procédé décrit dans le schéma 2.

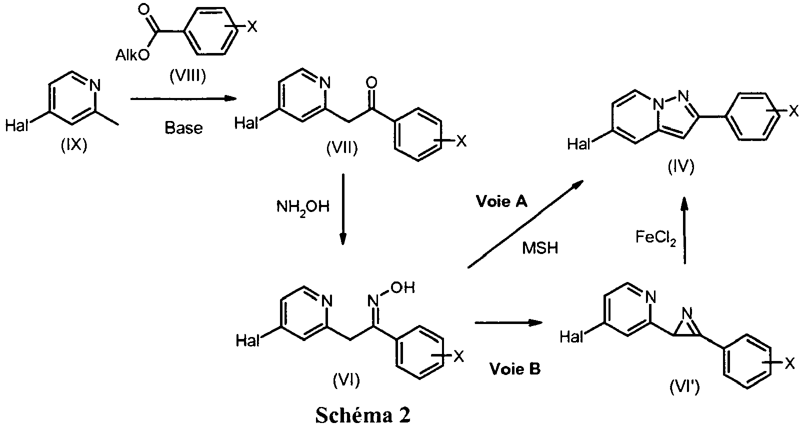

Dans le schéma 2 voie A, les composés de formule générale (IV) dans laquelle X est défini comme précédemment et Hal représente un atome d'halogène peuvent être préparés par action du *O*-(mésitylènesulfonyl)hydroxylamine (MSH) sur un composé de formule générale (VI) dans laquelle X et Hal sont définis comme précédemment, par exemple selon la méthode décrite par Y. Tamura, J.-H. Kim, Y. Miki, H. Hayashi, M. Ikeda, dans J.Het. Chem., 1975, 12, 481.

Dans le schéma 2 voie B, on peut également préparer les composés de formule générale (IV) dans laquelle X est défini comme précédemment et Hal représente un atome d'halogène par transformation des composés de formule générale (VI) en composés de formule générale (VI') dans laquelle X et Hal sont définis comme précédemment par action d'un anhydride d'acide tel que l'anhydride trifluoracétique en présence d'une base telle que la triéthylamine, puis cyclisation en composés de formule générale (IV) en présence d'un catalyseur comme le chlorure ferreux, par exemple selon la méthode décrite par K.S. Gudmundsson dans Bioorg. Med. Chem., 2005, 13, 5346.

Les composés (VI) peuvent être obtenus par action de l'hydroxylamine sur les composés (VII).

Les composés (VII) peuvent être obtenus à partir des composés (IX) par action des esters de formule générale (VIII) dans laquelle X est défini comme précédemment et Alk représente un groupement alkyle, en présence d'une base forte, par exemple selon la méthode décrite par K.S. Gudmundsson dans Bioorg. Med. Chem., 2005, 13, 5346.

Dans les schémas 1 et 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les énantiomères des composés de formule (I) peuvent être obtenus par dédoublement des racémiques, par exemple, par chromatographie sur colonne chirale du type CHIRALPAK AD 20µm par élution avec des solvants de type hydrocarbure (heptane par exemple) et alcools (méthanol et éthanol par exemple).

Les exemples suivants décrivent la préparation de certains composés conformes à l'invention. Ces exemples ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 : 1-{3-[2-(4-chlorophényl)pyrazolo[1,5-a]pyridin-5-yl]phényl}prop-2-yn-1-ol (composé 1 du tableau)

### 1.1 2-(4-bromopyridin-2-yl)-1-(4-chlorophényl)éthanone

Sous courant d'azote, 5 g (29,07 mmol) de 4-bromo-2-méthylpyridine et 11,27 g (61,04 mmol) de 4-chlorobenzoate d'éthyle sont placés dans un ballon et dissous dans 50 ml de tétrahydrofurane anhydre. On refroidit à 5°C et on ajoute goutte à goutte 70 mL (70 mmol) d'une solution d'hexaméthyldisilazane de lithium (1M dans le tétrahydrofurane). Après addition, le mélange est agité à température ambiante pendant 2h, refroidi à 5°C, puis 100 mL d'eau sont additionnés progressivement. Le milieu est ensuite dilué avec 250 mL d'acétate d'éthyle et 100 mL d'eau. La phase organique est séparée, la phase aqueuse est extraite deux fois avec 100 ml d'acétate d'éthyle. Les phases organiques sont ensuite réunies, séchées sur sulfate de sodium et filtrées. On ajoute ensuite au filtrat 15 g de silice, concentre sous pression réduite. La poudre obtenue est utilisée comme dépôt solide pour une chromatographie sur gel de silice avec pour éluant un mélange de cyclohexane et d'acétate d'éthyle (9/1). 8,4 g (93%) de composé sont obtenus sous forme d'une poudre jaune.
LC-MS : M+H = 310
RMN-¹H (DMSO) δ (ppm) : 4,6 (s, 2H) ; 6,4 (s, 1H) ; 7,4 (s, 1H) ; de 7,5 à 7,6 (m, 6H) ; 7,7 (s, 1H) ; 7,9 (d, 2H) ; 8,1 (d,2H) ; 8,3 (d, 1H) ; 8,4 (d, 1H) ; 15,0 (s, 1 H) (mélange *cétone* / *énol:* 40 / 60).

### 1.2 2-(4-Bromopyridin-2-yl)-1-(4-chlorophényl)éthanone oxime

On place dans un ballon 8,4 g (27,05 mmol) de 2-(4-bromopyridin-2-yl)-1-(4-chlorophényl)éthanone dans 150 mL d'éthanol. On ajoute 22 mL (272,56 mmol) de pyridine et 7,5 g (107,93 mmol) d'hydroxylamine monochlorhydrate. Le mélange est ensuite agité pendant 5 heures à température ambiante puis le milieu réactionnel est concentré sous pression réduite jusqu'à obtention d'un solide pâteux jaune que l'on reprend avec 400mL d'acétate d'éthyle et 400 mL d'eau. La phase organique est séparée, la phase aqueuse est extraite trois fois avec 200 mL d'acétate d'éthyle. Les phases organiques sont ensuite réunies, séchées sur sulfate de sodium et filtrées. Le filtrat est concentré sous pression réduite : on obtient 8,1 g (91,9%) de composé sous forme d'une poudre bleue.
LC-MS : M+H = 325
RMN-¹H (DMSO) δ (ppm) : 4,3 (s, 2H) ; 7,45 (m, 2H) ; 7,50 (d, 1H) ; 7,55 (s, 1H) ; 7,75 (m, 2H) ; 8,35 (d, 1H) ; 11,65 (s, 1H).

### 1.3. 5-bromo-2-(4-chlorophényl)pyrazolo[1,5-a]pyridine

On place dans un ballon 12,9 g (45,21 mmol) de *O*-(2-mésitylènesulfonyl)acétohydroxamate d'éthyle dans 30 mL de 1,4-dioxane. On refroidit à 0°C et on ajoute 13,5 mL (156,60 mmol) d'acide perchlorique (70% dans l'eau). On ajoute ensuite 10 mL de 1,4-dioxane puis le milieu est agité vigoureusement pendant 2h30 minutes à 0°C. Le milieu est ensuite versé dans 350mL d'eau glacée. On laisse le milieu vers 0°C. pendant 10 minutes puis on récupère par filtration sur verre fritté le solide blanc formé (ne pas sécher totalement, le produit est potentiellement explosif à l'état sec). Le solide pâteux blanc obtenu est lavé avec 350 mL d'eau glacée puis est repris avec 250 mL de 1,2-dichloroéthane et 150 mL de saumure refroidie vers 5°C. On récupère la phase organique que l'on filtre sur cartouche hydrophobe. On récupère le filtrat que l'on ajoute goutte à goutte sur une solution refroidie vers 0°C de 8,1g (24,88 mmol) de 2-(4-bromopyridin-2-yl)-1-(4-chlorophényl)éthanone oxime (composé obtenu dans l'étape 1.2) dans 150mL de 1,2-dichloroéthane.

Après l'ajout, on laisse revenir et on agite à température ambiante pendant 3 heures. On ajoute ensuite successivement au milieu 250 mL de dichlorométhane, 200mL d'eau et 100 mL d'une solution aqueuse NaOH (1N). On laisse agiter puis on décante. La phase organique est séparée et la phase aqueuse est extraite avec 2 fois 200 mL de dichlorométhane. Les phases organiques sont ensuite réunies, filtrées sur cartouche hydrophobe (Colonne d'extraction liquide/liquide 70 mL, Radleys®) puis mélangées avec 15 g de silice. Le filtrat est ensuite concentré sous pression réduite : on obtient une poudre marron que l'on utilise comme dépôt solide pour une chromatographie sur gel de silice en éluant avec un mélange de cyclohexane et de dichlorométhane (1/1). On obtient 5,8 g (75%) de composé sous forme d'un solide cotonneux légèrement jaune.
LC-MS : M+H = 307.
RMN-¹H (DMSO) δ (ppm) : 7,0 (d, 1H) ; 7,1 (s, 1H) ; 7,6 (d, 2H) ; 8,0 (s, 1H) ; 8,1 (d, 2H) ; 8,7 (d, 1H).

### 1.4 3-[2-(4-chlorophényl)pyrazolo[1,5-a]pyridin-5-yl]benzaldéhyde

Sous courant d'azote, sont introduits 0,300 g (0,98 mmol) de 5-bromo-2-(4-chlororophényl)pyrazolo[1,5-*a*]pyridine obtenu selon le protocole décrit en 1.3, 0,292 g (1,95 mmol) d'acide 3-formylphénylboronique, 0,94 g (2,88 mmol) de carbonate de césium dans 5 mL d'un mélange 9/1 de tétrahydrofurane et d'eau. 0,08 g (0,10 mmol) de [1,1'-bis(diphénylphosphino)ferrocène]dichloropalladium (II) sont ajoutés et le milieu est chauffé à 70°C pendant 2 heures. Le milieu est ensuite remis à température ambiante puis dilué avec du dichlorométhane et de l'eau. On filtre le milieu sur cartouche hydrophobe (Colonne d'extraction liquide/liquide 70 mL, Radleys®), récupère la phase organique à laquelle on ajoute 2 g de silice. Après évaporation du solvant, on purifie le résidu par chromatographie sur gel de silice en éluant avec un mélange de cyclohexane et d'acétate d'éthyle (9/1). On obtient 0,302 g (93%) de produit attendu sous la forme d'une poudre jaune.
Point de fusion (°C) : 152-154
LC-MS : M+H = 333
RMN-¹H (DMSO) δ (ppm) : 7,2 (s, 1H) ; 7,35 (d, 1H) ; 7,55 (d, 2H) ; 7,75 (t, 1H) ; 7,95 (d, 1H) ; 8,05 (d, 2H) ; 8,15 (s, 1H) ; 8,20 (d, 1H) ; 8,40 (s, 1H) ; 8,85 (d, 1H) ; 10,15 (s, 1H).

### 1.5 1-{3-[2-(4-chlorophényl)pyrazolo[1,5-a]pyridin-5-yl]phényl}prop-2-yn-1-ol

Sous courant d'azote, 0,100 g (0,30 mmol) de 3-[2-(4-chlorophényl)pyrazolo[1,5-*a*]pyridin-5-yl]benzaldéhyde obtenus selon 1.4 sont mis en solution dans 8 mL de tétrahydrofurane. Le milieu est refroidi à 0°C pour addition lente de 0,800 mL (0,40mmol) d'une solution de bromure d'éthynylmagnésium (0,5 M dans le tétrahydrofurane). Le milieu est ensuite remis à température ambiante puis agité pendant 45 minutes. On hydrolyse en ajoutant à froid et au goutte à goutte une solution aqueuse saturée en chlorure d'ammonium, puis dilue avec 40 mL d'acétate d'éthyle et 30 mL d'eau. La phase organique est récupérée et la phase aqueuse est extraite 2 fois avec 20 mL d'acétate d'éthyle. Les phases organiques sont ensuite réunies, puis concentrées sous pression réduite après avoir ajouté 1 g de silice. Le résidu est ensuite purifié par chromatographie sur gel de silice en éluant le dépôt solide avec un mélange de cyclohexane et d'acétate d'éthyle (9/1). On obtient 0,02 g (18 %) de produit attendu sous la forme d'une poudre beige.
Point de fusion (°C) : 195-197.
LC-MS : M+H = 359
RMN-¹H (DMSO) δ (ppm) : 3,55 (s, 1H) ; 5,50 (m, 1H) ; 6,15 (d, 1H) ; 7,20 (s, 1H) ; 7,30 (d, 1 H) ; 7,60 (m, 4H) ; 7,80 (m, 1 H) ; 7,90 (s, 1 H) ; 8,00 (m, 3H) ; 8,80 (d, 1H).

### Exemple 2 : 1-{3-[2-(4-fluorophényl)pyrazolo[1,5-a]pyridin-5-yl]phényl}prop-2-yn-1-ol (composé 2 du tableau)

### 2.1 2-(4-bromopyridin-2-yl)-1-(4-fluorophényl)éthanone

Sous courant d'azote, 5,0 g (29,07 mmol) de 4-bromo-2-pyridine et 10,2 g (60,95 mmol) de 4-fluorobenzoate d'éthyle sont placés dans un ballon et dissous dans 50 mL de tétrahydrofurane anhydre. On refroidit à 0°C et on ajoute goutte à goutte 70 mL (70 mmol) d'une solution d'hexaméthyldisilazane de lithium (1M dans le tétrahydrofurane). Après addition, le mélange est agité à température ambiante pendant 2h, avant d'être refroidi à 5°C et d'ajouter progressivement, 100 mL d'eau au milieu. Le milieu est ensuite dilué avec 250 mL d'acétate d'éthyle et 100 mL d'eau. La phase organique est séparée et la phase aqueuse est extraite deux fois avec 100 mL d'acétate d'éthyle. Les phases organiques sont ensuite réunies, séchées sur sulfate de sodium et filtrées. On ajoute ensuite au filtrat 15 g de silice avant de le concentrer sous pression réduite. La poudre obtenue est utilisée comme dépôt solide pour une chromatographie sur gel de silice avec pour éluant un mélange de cyclohexane et d'acétate d'éthyle (9/1). 7,5 g (88%) de composé sont obtenus sous forme d'une poudre jaune.
LC-MS : M+H = 294 (ratio *cétone* / *énol:* 43 / 57)
RMN-¹H (DMSO) δ (ppm) : 4,56 (s, 2H) ; 6,34 (s, 1H) ; de 7,23 à 7,40 (m, 5H) ; 7,53 (d, 1H) ; 7,56 (m, 1H) ; 7,70 (d, 1H) ; de 7,81 à 7,92 (m, 2H) ; de 8,04 à 8,16 (m,2H) ; 8,29 (d, 1H) ; 8,37 (d, 1H) ; 15,0 (s, 1H).

### 2.2 2-(4-bromopyridin-2-yl)-1-(4-fluorophényl)éthanone oxime

7,5 g (24,26 mmol) de 2-(4-bromopyridin-2-yl)-1-(4-fluorophényl)éthanone sont placés dans un ballon contenant 100 mL d'éthanol absolu. 20 mL (247,78 mmol) de pyridine et 7,08 g (101,88 mmol) d'hydroxylamine monochlorhydrate sont ajoutés avant de laisser agiter le milieu pendant 3h à température ambiante. L'éthanol est ensuite évaporé sous vide et le résidu obtenu est repris avec 250 mL d'eau et 250 mL d'acétate d'éthyle. La phase organique est séparée, puis on extrait la phase aqueuse 5 fois avec 150 mL d'acétate d'éthyle. Les phases organiques sont ensuite réunies, séchées sur sulfate de sodium et concentrées sous vide. 7,82 g de composé sont obtenus.
LC-MS : M+H = 309.
RMN-¹H (DMSO) δ (ppm): 4,26 (s, 2H) ; 7,19 (t, 2H) ; 7,50 (m, 2H) ; 7,75 (m, 2H) ; 8,33 (d, 1H) ; 11,50 (s, 1H). (obtention de l'oxime (E)).

### 2.3 5-bromo-2-(4-fluororophényl)pyrazolo[1,5-a]pyridine

7,82 g (25,50 mmol) de 2-(4-bromopyridin-2-yl)-1-(4-fluorophényl)éthanone oxime sont placés dans un ballon et dissous dans 400 mL de 1,2-dichloroéthane. 170 mL d'une solution de *O*-(mésitylènesulfonyl)hydroxylamine (0,27 M dans le 1,2-dichloroéthane - composé obtenu selon le protocole décrit en 1.3) est ajoutée goutte à goutte au milieu refroidi vers 0°C. Après l'ajout, le milieu est agité à température ambiante pendant 1h30. Le milieu est ensuite dilué avec 200 mL d'eau et 200 mL d'une solution de soude (1N). Le milieu biphasique est agité puis décanté. La phase organique est séparée, puis la phase aqueuse est extraite 4 fois avec 200 mL de dichlorométhane. Les phases organiques sont ensuite réunies, séchées sur sulfate de sodium et filtrées. On ajoute ensuite au filtrat 15 g de silice avant de le concentrer sous pression réduite. La poudre obtenue est utilisée comme dépôt solide pour une chromatographie sur gel de silice avec pour éluant un mélange de cyclohexane et de dichlorométhane (1/1). 5,06 g (68%) de composé sont obtenus sous forme d'une poudre cotonneuse blanche.
LC-MS : M+H = 291.
RMN-¹H (DMSO) δ (ppm): de 7,00 à 7,10 (m, 2H) ; 7,45 (m, 2H) ; 8,05 (m, 3H) ; 8,70 (d, 1H).

### 2.4 3-[2-(4-fluorophényl)pyrazolo[1,5-a]pyridin-5-yl]benzaldéhyde

Sous courant d'azote sont introduits 0,200 g (0,69 mmol) de 5-bromo-2-(4-fluorophényl)pyrazolo[1,5-*a*]pyridine obtenu à l'étape 2.3, 0,125 g (0,83 mmol) d'acide 3-formylphénylboronique, 0,670 g (2,06 mmol) de carbonate de césium dans 5 mL d'un mélange 9/1 de tétrahydrofurane et d'eau. 0,055 g (0,07 mmol) de [1,1'-bis(diphénylphosphino)ferrocène]dichloropalladium (II) sont ajoutés et le milieu est chauffé à 70°C pendant 3 heures. On rajoute ensuite 0,063 g (0,42 mmol) d'acide 3-formylphénylboronique, 0,335 g (1,03 mmol) de carbonate de césium et 0,028 g (0,03 mmol) de [1,1'-bis(diphénylphosphino)ferrocène]dichloropalladium (II) en laissant agiter à 70°C pendant 4h. Le milieu est ensuite remis à température ambiante puis dilué avec du dichlorométhane et de l'eau. On filtre le milieu biphasique sur cartouche hydrophobe (Colonne d'extraction liquide/liquide 70 mL, Radleys®), récupère la phase organique à laquelle on ajoute 1,2 g de silice. Après évaporation du solvant, on purifie le résidu par chromatographie sur gel de silice en éluant avec un mélange de cyclohexane et d'acétate d'éthyle (8/2). On obtient 0,175g (80%) de produit attendu sous la forme d'une poudre blanche.
LC-MS : M+H = 317
RMN-¹H (DMSO) δ (ppm) : 7,15 (s, 1H) ; de 7,30 à 7,37 (m, 3H) ; 7,77 (t,1H) ; 7,98 (m, 1H) ; 8,08 (m, 2H) ; 8,15 (m, 1H) ; 8,20 (m, 1H) ; 8,38 (s, 1H) ; 8,82 (d, 1H) ; 10,15 (s,1H).

### 2.5 1-{3-[2-(4-fluorophényl)pyrazolo[1,5-a]pyridin-5-yl]phényl}prop-2-yn-1-ol

Sous courant d'azote 0,100 g (0,32 mmol) de 3-[2-(4-fluorophényl)pyrazolo[1,5-*a*]pyridin-5-yl]benzaldéhyde obtenus à l'étape 2.4 sont mis en solution dans 8 mL de tétrahydrofurane. Le milieu est refroidi à 5°C pour addition lente d'une solution de 2 mL (1 mmol) de bromure d'éthynylmagnésium (0,5M dans le tetrahydrofurane). Le milieu est remis progressivement à température ambiante puis agité pendant 30 minutes. On hydrolyse en ajoutant à froid et au goutte à goutte une solution aqueuse saturée en chlorure d'ammonium, puis on dilue le milieu avec 50 mL de dichlorométhane et 40 mL d'eau. Le milieu est ensuite filtré sur cartouche hydrophobe (Colonne d'extraction liquide/liquide 70 mL, Radleys®), on récupère la phase organique à laquelle on ajoute 1 g de silice avant de la concentrer sous pression réduite. Le résidu est purifié par chromatographie sur gel de silice en éluant le dépôt solide avec un mélange de cyclohexane et d'acétate d'éthyle (7/3). On obtient 0,055g (50%) de produit attendu sous la forme d'une poudre blanche.
Point de fusion (°C) : 192-194.
LC-MS : M+H = 343
RMN-¹H (DMSO) δ (ppm) : 3,55 (s, 1H) ; 5,50 (m, 1H) ; 6,15 (d, 1H) ; 7,10 (s, 1H) ; 7,20 (d, 1H) ; 7,35 (m, 2H) ; 7,55 (q, 2H) ; 7,75 (q, 1H) ; 7,90 (s, 1H) ; 8,00 (s, 1H) ; 8,10 (m, 2H) ; 8,80 (d, 1 H).

### Exemple 3: 1-{3-[2-(4-Fluorophényl)pyrazolo[1,5-a]pyridin-5-yl]phényl}but-2-yn-1-ol (composé 3 du tableau)

On procède suivant le mode opératoire décrit dans l'étape 2.5. A partir de 0,300 g (0,95 mmol) de 3-[2-(4-fluorophényl)pyrazolo[1,5-*a*]pyridin-5-yl]benzaldéhyde obtenu selon le procédé décrit en 2.4, en solution dans 10 mL de tétrahydrofurane, puis addition de 5,70 mL d'une solution (2,85 mmol) de bromure de propyn-1-ylmagnésium (0,5M dans le tetrahydrofurane) et après chromatographie sur gel de silice en éluant avec un mélange de cyclohexane et d'acétate d'éthyle (8/2), on obtient 0,23 g (67 %) de produit attendu sous la forme d'une poudre jaune pâle.
Point de fusion (°C) : 164-166.
LC-MS : M+H = 357
RMN-¹H (DMSO) δ (ppm) : 1,87 (s, 3H) ; 5,45 (m, 1H) ; 5,95 (d, 1H) ; 7,12 (s, 1H) ; 7,25 (dd, 1H) ; 7,32 (t, 2H) ; 7,53 (m, 2H) ; 7,75 (m, 1H) ; 7,86 (s, 1H) ; 7,99 (m, 1H) ; 8,08 (m, 2H) ; 8,78 (d, 1H).

### Exemple 4: 2-{3-[2-(4-Fluorophényl)pyrazolo[1,5-a]pyridin-5-yl]phényl}but-3-yn-2-ol (composé 4 du tableau)

### 4.1 1-{3-[2-(4-Fluorophényl)pyrazolo[1,5-a]pyridin-5-yl]phényl}éthanone

On procède suivant le mode opératoire décrit dans l'étape 2.4. A partir de 0,300 g (1,03 mmol) de 5-bromo-2-(4-fluorophényl)pyrazolo[1,5-*a*]pyridine obtenu à l'étape 2.3, 0,185 g (1,13 mmol) d'acide 3-acétylphénylboronique, 1,00 g (3,09 mmol) de carbonate de césium et 0,084 g (0,10 mmol) de [1,1'-bis(diphénylphosphino)ferrocène]dichloropalladium (II) dans 5 mL d'un mélange 9/1 de tétrahydrofurane et d'eau. Après chromatographie sur gel de silice en éluant avec un mélange de cyclohexane et d'acétate d'éthyle (8/2), on obtient 0,268 g (78 %) de produit attendu sous la forme d'une poudre beige.
RMN-¹H (DMSO) δ (ppm) : 2,70 (s, 3H) ; 7,10 (s, 1H) ; 7,32 (m, 3H) ; 7,68 (m, 1H) ; de 7,95 à 8,12 (m, 5H) ; 8,32 (m, 1H) ; 8,80 (d, 1H).

### 4.2 2-{3-[2-(4-Fluorophényl)pyrazolo[1,5-a]pyridin-5-yl]phényl}but-3-yn-2-ol

On procède suivant le mode opératoire décrit dans l'étape 2.5. A partir de 0,261 g (0,79 mmol) de 1-{3-[2-(4-fluorophényl)pyrazolo[1,5-*a*]pyridin-5-yl]phényl}éthanone obtenu à l'étape 4.1 en solution dans 10 mL de tétrahydrofurane, puis addition de 4,74 mL d'une solution (2,37 mmol) de bromure d'éthynylmagnésium (0,5M dans le tetrahydrofurane) et après chromatographie sur gel de silice en éluant avec un mélange de cyclohexane et de dichlorométhane (1/9), on obtient 0,018 g (6%) de produit attendu sous la forme d'une poudre blanche.
Point de fusion (°C) : 215-217.
LC-MS : M+H = 357
RMN-¹H (DMSO) δ (ppm) : 1,75 (s, 3H) ; 3,58 (s, 1H) ; 6,20 (s, 1H) ; 7,12 (s, 1H) ; 7,25 (m, 1H) ; 7,32 (t, 2H) ; 7,53 (m, 1H) ; 7,65 (m, 1H) ; 7,75 (m, 1H) ; 7,98 (m, 2H) ; 8,08 (m, 2H) ; 8,80 (d, 1H).

### Exemple 5: 1-{2-[2-(4-Fluorophényl)pyrazolo[1,5-a]pyridin-5-yl]phényl}prop-2-yn-1-ol (composé 5 du tableau)

### 5.1 2-[2-(4-Fluorophényl)pyrazolo[1,5-a]pyridin-5-yl]benzaldéhyde

On procède suivant le mode opératoire décrit dans l'étape 2.4. A partir de 0,300 g (1,03 mmol) de 5-bromo-2-(4-fluorophényl)pyrazolo[1,5-*a*]pyridine obtenu à l'étape 2.3, 0,170 g (1,13 mmol) d'acide 2-formylphénylboronique, 1,00 g (3,09 mmol) de carbonate de césium et 0,084 g (0,10 mmol) de [1,1'-bis(diphénylphosphino)ferrocène]dichloropalladium (II) dans 5 mL d'un mélange 9/1 de tétrahydrofurane et d'eau. Après chromatographie sur gel de silice en éluant avec un mélange de cyclohexane et d'acétate d'éthyle (85/15), on obtient 0,254 g (77 %) de produit attendu sous la forme d'une poudre beige.
LC-MS: M+H = 317
RMN-¹H (DMSO) δ (ppm) : 7,05 (d, 1H) ; 7,12 (s, 1H) ; 7,35 (t, 2H) ; 7,78 (m, 2H) ; 7,78 (s, 1H) ; 7,81 (m, 1H) ; 8,00 (d, 1H) ; 8,05 (m, 2H) ; 8,80 (d, 1H), 10,05 (s, 1H).

### 5.2 1-{2-[2-(4-Fluoro-phényl)pyrazolo[1,5-a]pyridin-5-yl]phényl}prop-2-yn-1-ol

On procède suivant le mode opératoire décrit dans l'étape 2.5. A partir de 0,245 g (0,77 mmol) de 2-[2-(4-fluorophényl)pyrazolo[1,5-*a*]pyridin-5-yl]benzaldéhyde obtenu à l'étape 5.1 en solution dans 15 mL de tétrahydrofurane, puis addition de 4,65 mL d'une solution (2,32 mmol) de bromure d'éthynylmagnésium (0,5M dans le tetrahydrofurane) et après chromatographie sur gel de silice en éluant avec du dichlorométhane, on obtient 0,08 g (30%) de produit attendu sous la forme d'une poudre jaune pâle.
Point de fusion (°C) : 104-106.
LC-MS : M+H = 343
RMN-¹H (DMSO) δ (ppm) : 3,50 (s, 1H) ; 5,35 (m, 1H) ; 6,12 (m, 1H) ; 6,95 (dd, 1H) ; 7,12 (s, 1H) ; de 7,30 à 7,40 (m, 3H) ; 7,46 (m, 1H) ; 7,52 (m, 1H) ; 7,69 (m, 1H) ; 7,83 (m, 1H) ; 8,07 (m, 2H) ; 8,78 (d, 1H).

### Exemple 6: 1-{2,6-Difluoro-3-[2-(4-fluorophényl)pyrazolo[1,5-a]pyridin-1-yl]phényl} prop-2-yn-1-ol (composé 6 du tableau)

### 6.1 2,6-Difluoro-3-[2-(4-fluorophényl)pyrazolo[1,5-a]pyridin-5-yl]benzaldéhyde

On procède suivant le mode opératoire décrit dans l'étape 2.4. A partir de 0,300 g (1,03 mmol) de 5-bromo-2-(4-fluorophényl)pyrazolo[1,5-*a*]pyridine obtenu à l'étape 2.3, 0,210 g (1,13 mmol) d'acide 2,4-difluoro-3-formylphénylboronique, 1,00 g (3,09 mmol) de carbonate de césium et 0,084 g (0,10 mmol) de [1,1'-bis(diphénylphosphino)ferrocène]dichloropalladium (II) dans 5 mL d'un mélange 9/1 de tétrahydrofurane et d'eau. Après chromatographie sur gel de silice en éluant avec un mélange de cyclohexane et d'acétate d'éthyle (8/2), on obtient 0,278 g (76 %) de produit attendu sous la forme d'une poudre beige.
LC-MS : M+H = 353
RMN-¹H (DMSO) δ (ppm) : 7,12 (m, 1H) ; 7,20 (s, 1H) ; de 7,30 à 7,50 (m, 3H) ; 7,95 (m, 1H) ; de 8,05 à 8,15 (m, 3H) ; 8,85 (d, 1H), 10,32 (s, 1H).

### 6.2 1-{2,6-Difluoro-3-[2-(4-fluorophényl)pyrazolo[1,5-a]pyridin-5-yl]phényl}prop-2-yn-1-ol

On procède suivant le mode opératoire décrit dans l'étape 2.5. A partir de 0,270 g (0,77 mmol) de 2,6-difluoro-3-[2-(4-fluorophényl)pyrazolo[1,5-*a*]pyridin-5-yl]benzaldéhyde obtenu à l'étape 6.1 en solution dans 15 mL de tétrahydrofurane, puis addition de 4,60 mL (2,30 mmol) d'une solution de bromure d'éthynylmagnésium (0,5M dans le tetrahydrofurane) et après chromatographie sur gel de silice en éluant avec un mélange de cyclohexane et de dichlorométhane (1/3), on obtient 0,171 g (57%) de produit attendu sous la forme d'une poudre jaune pâle.
Point de fusion (°C) : 151-153.
LC-MS : M+H = 379
RMN-¹H (DMSO) δ (ppm) : 3,50 (s, 1H) ; 5,75 (s, 1H) ; 6,28 (d, 1H) ; 7,05 (m, 1H) ; 7,15 (s, 1H) ; de 7,25 à 7,38 (m, 3H) ; 7,70 (m, 1H) ; 7,85 (s, 1H) ; 8,05 (m, 2H) ; 8,78 (d, 1H).

### Exemple 7: 1-{3-[2-(2-Fluorophényl)pyrazolo[1,5-a]pyridin-5-yl]phényl}prop-2-yn-1-ol (composé 7 du tableau)

### 7.1 5-Bromo-2-(2-fluoro-phenyl)-pyrazolo[1,5-a]pyridine

Sous courant d'azote, 1 g (5,81 mmol) de 4-bromo-2-méthylpyridine et 1,88 g (12,20 mmol) de 2-fluorobenzoate de méthyle sont placés dans un ballon et dissous dans 25 mL de tétrahydrofurane anhydre. On refroidit à 5°C et on ajoute goutte à goutte 14 mL (14 mmol) d'une solution d'hexaméthyldisilazane de lithium (1M dans le tétrahydrofurane). Après addition, le mélange est agité à température ambiante pendant 2h30, refroidi à 5°C, puis 15 mL d'eau sont additionnés progressivement. Le milieu est ensuite dilué avec 100 mL d'acétate d'éthyle et 100 mL d'eau. La phase organique est séparée, la phase aqueuse est extraite avec 100 mL d'acétate d'éthyle. Les phases organiques sont ensuite réunies, séchées sur sulfate de sodium et filtrées. On ajoute ensuite au filtrat 6 g de silice, concentre sous pression réduite. La poudre obtenue est utilisée comme dépôt solide pour une chromatographie sur gel de silice avec pour éluant un mélange de cyclohexane et d'acétate d'éthyle (95/5). On récupère 1,71 g d'une poudre jaune que l'on place dans un ballon en présence de 1,60g (23,02 mmol) d'hydroxylamine chlorhydrate, 5 mL (61,95 mmol) de pyridine et 30 mL d'éthanol. On laisse agiter le milieu à température ambiante pendant toute une nuit puis on concentre sous pression réduite le milieu réactionnel. Le résidu obtenu est repris avec 50 mL d'acétate d'éthyle et 50 mL d'eau. On récupère la phase organique puis on extrait la phase aqueuse avec 50 mL d'acétate d'éthyle. Les phases organiques sont ensuite réunies, séchées sur sulfate de sodium puis concentrées sous pression réduite. 1,05 g d'une cire orange sont obtenus que l'on place dans un ballon contenant 50 mL de 1,2-dichloroéthane. Le milieu est alors refroidi vers 5°C puis on ajoute goutte à goutte 61 mL d'une solution de *O*-(mésitylènesulfonyl)hydroxylamine dans du 1,2-dichloroéthane (C=0,11 mol/L). Le milieu est agité une nuit à t.a. puis on ajoute 50 mL d'eau, 50 mL d'une solution aqueuse NaOH (1N) et 100 mL de dichlorométhane. La phase organique est récupérée puis la phase aqueuse est extraite avec 100 mL de dichlorométhane. Les phases organiques sont ensuite réunies, séchées sur sulfate de sodium et filtrées. On ajoute ensuite au filtrat 5 g de silice, puis on concentre sous pression réduite. La poudre obtenue est utilisée comme dépôt solide pour une chromatographie sur gel de silice avec pour éluant un mélange de cyclohexane et d'acétate d'éthyle (8/2). On récupère 0,675 g (39,9%) de produit attendu sous forme d'une poudre jaune.
LC-MS : M+H = 291
RMN-¹H (DMSO) δ (ppm) : 7,03 (d, 1H) ; 7,1 (dd, 1H) ; de 7,33 à 7,43 (m, 2H) ; de 7,48 à 7,55 (m, 1H) ; de 8,05 à 8,18 (m, 2H) ; 8,73 (d, 1H).

### 7.2 3-[2-(2-Fluorophényl)pyrazolo[1,5-a]pyridin-5-yl]benzaldéhyde

On procède suivant le mode opératoire décrit dans l'étape 2.4. A partir de 0,150 g (0,52 mmol) de 5-bromo-2-(2-fluorophényl)pyrazolo[1,5-*a*]pyridine obtenu à l'étape 7.1, 0,092 g (0,61 mmol) d'acide 3-formylphénylboronique, 0,502 g (1,54 mmol) de carbonate de césium et 0,042 g (0,050 mmol) de [1,1'-bis(diphénylphosphino)ferrocène]dichloropalladium (II) dans 5 mL d'un mélange 9/1 de tétrahydrofurane et d'eau. Après chromatographie sur gel de silice en éluant avec un mélange de cyclohexane et d'acétate d'éthyle (8/2), on obtient 0,130 g (79 %) de produit attendu sous la forme d'une poudre beige.
LC-MS : M+H = 317
RMN-¹H (DMSO) δ (ppm) : 7,10 (m, 1H) ; de 7,35 à 7,45 (m, 3H) ; 7,50 (m, 1H) ; 7,78 (t, 1 H) ; 7,98 (m, 1H) ; de 8,15 à 8,25 (m, 3H) ; 8,38 (s, 1H) ; 8,88 (d, 1H) ; 10,15 (s, 1H).

### 7.3 1-{3-[2-(2-Fluorophényl)pyrazolo[1,5-a]pyridin-5-yl]phényl}prop-2-yn-1-ol

On procède suivant le mode opératoire décrit dans l'étape 2.5. A partir de 0,125 g (0,40 mmol) de 3-[2-(2-fluorophényl)pyrazolo[1,5-*a*]pyridin-5-yl]benzaldéhyde obtenu à l'étape 7.2 en solution dans 15 mL de tétrahydrofurane, puis addition de 3,50 mL (1,75 mmol) d'une solution de bromure d'éthynylmagnésium (0,5M dans le tetrahydrofurane) et après chromatographie sur gel de silice en éluant avec un mélange de cyclohexane et d'acétate d'éthyle (8/2), on obtient 0,112 g (82%) de produit attendu sous la forme d'une poudre beige.
Point de fusion (°C) : 157-159.
LC-MS : M+H = 343
RMN-¹H (DMSO) δ (ppm) : 3,55 (d, 1H) ; 5,55 (m, 1H) ; 6,15 (m, 1H) ; 7,12 (m, 1H) ; 7,30 (m, 1H) ; de 7,32 à 7,45 (m, 2H) ; de 7,46 à 7,52 (m, 1H) ; 7,55 (m, 2H) ; 7,78 (m, 1H) ; 7,90 (s, 1H) ; 8,08 (s, 1H) ; 8,15 (m, 1H) ; 8,82 (d, 1H).

### Exemple 8: 1-[3-(2-p-Tolylpyrazolo[1,5-a]pyridin-5-yl)phéyl]prop-2-yn-1-ol (composé 8 du tableau)

### 8.1 2-(4-Bromo-pyridin-2-yl)-1-p-tolyléthanone

Sous courant d'azote, 1 g (5,81 mmol) de 4-bromo-2-méthylpyridine et 1,75 g (11,60 mmol) de 4-méthylbenzoate de méthyle sont placés dans un ballon et dissous dans 30 mL de tétrahydrofurane anhydre. On refroidit à 5°C et on ajoute goutte à goutte 14 mL (14 mmol) d'une solution d'hexaméthyldisilazane de lithium (1M dans le tétrahydrofurane). Après addition, le mélange est agité à température ambiante pendant 2h30, puis refroidi à 5°C, avant d'ajouter progressivement 20 mL d'eau. Le milieu est ensuite dilué avec 200 mL d'acétate d'éthyle et 200 mL d'eau. La phase organique est séparée, séchée sur sulfate de sodium et filtrée. On ajoute ensuite au filtrat 5 g de silice avant le concentrer sous pression réduite. La poudre obtenue est utilisée comme dépôt solide pour une chromatographie sur gel de silice avec pour éluant un mélange de cyclohexane et d'acétate d'éthyle (95/5), on obtient 1,03 g (61%) de composé sous forme d'une poudre jaune.
LC-MS : M+H = 290

### 8.2 4-Bromo-2-(3-p-tolyl-2H-azirin-2-yl)pyridine

On place 1,03 g de 2-(4-bromo-pyridin-2-yl)-1-p-tolyléthanone obtenu à l'étape 8.1 dans un ballon avec 0,99 g (14,2 mmol) d'hydroxylamine monochlorhydrate, 3mL (37 mmol) de pyridine et 100 mL d'éthanol. On laisse agiter toute une nuit puis on concentre sous pression réduite le milieu réactionnel. Le résidu obtenu est alors repris avec 200 mL d'acétate d'éthyle et 200 mL d'eau. La phase organique est récupérée, séchée sur sulfate de sodium puis concentrée sous pression réduite. 1,10 g de composé sont récupérés et dissous dans un ballon contenant 0,660 mL (4,74 mmol) de triéthylamine et 30 mL de dichlorométhane. Le milieu réactionnel est ensuite refroidi vers 5°C puis 0,200 mL (1,42 mmol) d'anhydride de l'acide trifluoroacétique sont ajoutés goutte à goutte. Le milieu est ensuite agité à température ambiante pendant 3 heures avant d'être hydrolysé avec 100 mL d'eau. Le milieu est ensuite agité pendant 10 minutes avant d'être filtré sur cartouche hydrophobe (Colonne d'extraction liquide/liquide 70 mL, Radleys®). On ajoute ensuite au filtrat 1,2g de silice avant de le concentrer sous pression réduite. La poudre obtenue est utilisée comme dépôt solide pour une chromatographie sur gel de silice avec pour éluant un mélange de cyclohexane et d'acétate d'éthyle (95/5). On récupère 0,746 g (77%) de composé attendu sous forme d'une poudre blanche.
RMN-¹H (DMSO) δ (ppm) : 2,42 (d, 3H) ; 3,45 (s, 1H) ; de 7,42 à 7,58 (m, 4H) ; 7,78 (m, 2H) ; 8,30 (d, 1H).

### 8.3 5-Bromo-2-p-tolyl-pyrazolo[1,5-a]pyridine

0,746 g de 4-bromo-2-(3-p-tolyl-2H-azirin-2-yl)pyridine obtenue à l'étape 8.2 sont dissous en présence de 6,6 mg (0,052 mmol) de chlorure de fer (II) dans 30 mL de 1,2-diméthoxyéthane. Le milieu est alors porté à reflux pendant 6 heures. On rajoute ensuite 10 mg (0,078 mmol) de chlorure de fer (II) et on laisse ensuite agiter à nouveau à reflux pendant 3 heures. Le milieu est alors dilué avec 50 mL d'acétate d'éthyle et 50 mL d'eau. La phase organique est ensuite récupérée, séchée sur sulfate de sodium puis filtrée. On ajoute ensuite au filtrat 2g de silice avant de le concentrer sous pression réduite. La poudre obtenue est utilisée comme dépôt solide pour une chromatographie sur gel de silice avec pour éluant un mélange de cyclohexane et d'acétate d'éthyle (85/15). On récupère 0,534g (71%) de composé attendu sous forme d'une poudre jaune.
LC-MS : M+H = 287
RMN-¹H (DMSO) δ (ppm) : 2,48 (m, 3H) ; 7,00 (m, 2H) ; 7,32 (m, 2H) ; 7,88 (m, 2H) ; 8,00 (m, 1 H) ; 8,68 (d, 1 H).

### 8.4 3-(2-p-Tolylpyrazolo[1,5-a]pyridin-5-yl)benzaldéhyde

On procède suivant le mode opératoire décrit dans l'étape 2.4. A partir de 0,157 g (0,54 mmol) de 5-bromo-2-p-tolylpyrazolo[1,5-*a*]pyridine obtenu à l'étape 8.3, 0,098g (0,65 mmol) d'acide 3-formylphénylboronique, 0,535 g (1,64 mmol) de carbonate de césium et 0,045 g (0,055 mmol) de [1,1'-bis(diphénylphosphino)ferrocène]dichloropalladium (II) dans 5 mL d'un mélange 9/1 de tétrahydrofurane et d'eau. Après chromatographie sur gel de silice en éluant avec un mélange de cyclohexane et d'acétate d'éthyle (8/2), on obtient 0,110 g (65 %) de produit attendu sous la forme d'une poudre beige.
LC-MS : M+H = 313
RMN-¹H (DMSO) δ (ppm) : 2,38 (s, 3H) ; 7,12 (s, 1H) ; 7,32 (m, 3H) ; 7,78 (m, 1H) ; de 7,90 à 8 ,00 (m, 3H) ; 8,12 (m, 1H) ; 8,20 (m, 1H) ; 8,38 (s, 1H) ; 8,80 (d, 1H) ; 10,15 (s, 1H).

### 8.5 1-[3-(2-p-Tolylpyrazolo[1,5-a]pyridin-5-yl)phényl]prop-2-yn-1-ol

On procède suivant le mode opératoire décrit dans l'étape 2.5. A partir de 0,105 g (0,34 mmol) de 3-(2-p-tolylpyrazolo[1,5-*a*]pyridin-5-yl)benzaldéhyde obtenu à l'étape 8.4 en solution dans 15 mL de tétrahydrofurane, puis addition de 3,20 mL (1,60 mmol) d'une solution de bromure d'éthynylmagnésium (0,5M dans le tetrahydrofurane) et après chromatographie sur gel de silice en éluant avec un mélange de cyclohexane et d'acétate d'éthyle (8/2), on obtient 0,062 g (54%) de produit attendu sous la forme d'une poudre jaune.
Point de fusion (°C) : 183-185.
LC-MS : M+H = 339
RMN-¹H (DMSO) δ (ppm) : 2,40 (s, 3H) ; 3,55 (d, 1H) ; 5,55 (m, 1H) ; 6,15 (m, 1H) ; 7,10 (s, 1H) ; 7,22 (m, 1H) ; 7,32 (m, 2H) ; 7,55 (m, 2H) ; 7,78 (m, 1H) ; de 7,90 à 7,95 (m, 3H) ; 7,95 (s, 1H) ; 8,80 (d, 1H).

### Exemple 9 : (-)-1-{3-[2-(4-fluorophényl)pyrazolo[1,5-a]pyridin-5-yl]phényl}prop-2-yn-1-ol (composé 9 du tableau)

0,217 g de 1-{3-[2-(4-fluorophényl)pyrazolo[1,5-*a*]pyridin-5-yl]phényl}prop-2-yn-1-ol obtenu comme décrit dans l'exemple 2 sont injectés sur une colonne contenant 1200 g de phase stationnaire chirale Chiralpak AD 20 µm (8*35 cm). L'élution est effectuée à 140 mL puis 180 mL par minute avec pour éluant un mélange d'heptane, éthanol et méthanol (70/15/15). L'énantiomère (-)-1-{3-[2-(4-fluorophényl)pyrazolo[1,5-*a*]pyridin-5-yl]phényl}prop-2-yn-1-ol est élué en première position (temps de rétention = 60 min). Après concentration sous pression réduite, on obtient 0,106 g de produit attendu sous forme d'une poudre blanche.
Point de fusion (°C) : 147-149.
LC-MS : M+H = 343
RMN-¹H (DMSO) δ (ppm) : 3,55 (s, 1H) ; 5,50 (m, 1H) ; 6,15 (d, 1H) ; 7,10 (s, 1H) ; 7,20 (d, 1H) ; 7,35 (m, 2H) ; 7,55 (q, 2H) ; 7,75 (q, 1H) ; 7,90 (s, 1H) ; 8,00 (s, 1H) ; 8,10 (m, 2H) ; 8,80 (d, 1H).
Pouvoir rotatoire: α_{D} = - 6,9 ° (c=0,427 g/dL, DMSO, 589 nm)
Pureté énantiomérique : 99,5% (sur colonne CHIRALPAK AD-H 5 µm (250*4,6 mm) avec pour éluant un mélange d'hexane/éthanol/méthanol 70/15/15 (1mL/min), temps de rétention de 18,51 min)

### Exemple 10: (+)-1-{3-[2-(4-fluorophényl)pyrazolo[1,5-a]pyridin-5-yl]phényl}prop-2-yn-1-ol (composé 10 du tableau)

L'énantiomère (+)-1-{3-[2-(4-fluorophényl)pyrazolo[1,5-*a*]pyridin-5-yl]phényl}prop-2-yn-1-ol est élué en deuxième position (temps de rétention = 80 min) lors de la séparation effectuée dans l'exemple 9. Après concentration sous pression réduite, on obtient 0,098 g de produit attendu sous forme d'une poudre jaune claire.
Point de fusion (°C) : 139-141.
LC-MS : M+H = 343
RMN-¹H (DMSO) δ (ppm) : 3,55 (s, 1H) ; 5,50 (m, 1H) ; 6,15 (d, 1H) ; 7,10 (s, 1H) ; 7,20 (d, 1H) ; 7,35 (m, 2H) ; 7,55 (q, 2H) ; 7,75 (q, 1 H) ; 7,90 (s, 1H) ; 8,00 (s, 1H) ; 8,10 (m, 2H) ; 8,80 (d, 1H).
Pouvoir rotatoire: α_{D} = + 8,8 ° (c=0,502 g/dL, DMSO, 589 nm)
Pureté énantiomérique : 99% (sur colonne CHIRALPAK AD-H 5 µm (250*4,6 mm) avec pour éluant un mélange d'hexane/éthanol/méthanol 70/15/15 (1mL/min), temps de rétention de 24,58 min)

### Exemple 11 : (-)-2-{3-[2-(4-Fluorophényl)pyrazolo[1,5-a]pyridin-5-yl]phényl}but-3-yn-2-ol (composé 11 du tableau)

0,167 g de 2-{3-[2-(4-fluorophényl)pyrazolo[1,5-*a*]pyridin-5-yl]phényl}but-3-yn-2-ol obtenu comme décrit dans l'exemple 3 sont injectés sur une colonne contenant 1200 g de phase stationnaire chirale Chiralpak AD 20 µm.(8*35 cm) L'élution est effectuée à 250 mL par minute avec pour éluant un mélange d'heptane, éthanol et méthanol (70/15/15). L'énantiomère (-)-2-{3-[2-(4-fluorophényl)pyrazolo[1,5-*a*]pyridin-5-yl]phényl}but-3-yn-2-ol est élué en première position (temps de rétention = 32 min). Après concentration sous pression réduite, on obtient 0,082 g de produit attendu sous forme d'une poudre blanche.
Point de fusion (°C) : 122-124.
LC-MS : M+H = 357
RMN-¹H (DMSO) δ (ppm) : 1,87 (s, 3H) ; 5,45 (m, 1H) ; 5,95 (d, 1H) ; 7,12 (s, 1H) ; 7,25 (dd, 1H) ; 7,32 (t, 2H) ; 7,53 (m, 2H) ; 7,75 (m, 1H) ; 7,86 (s, 1H) ; 7,99 (m, 1H) ; 8,08 (m, 2H) ; 8,78 (d, 1H).
Pouvoir rotatoire: α_{D} = - 14,2 ° (c=0,323 g/dL, MeOH, 589 nm)
Pureté énantiomérique : 100% (sur colonne CHIRALPAK AD-H 5 µm (250*4,6 mm) avec pour éluant un mélange d'hexane/éthanol/méthanol 70/15/15 (1mL/min), temps de rétention de 15,00 min)

### Exemple 12 : (+)-2-{3-[2-(4-Fluorophényl)pyrazolo[1,5-a]pyridin-5-yl]phényl}but-3-yn-2-ol (composé 12 du tableau)

L'énantiomère (+)-2-{3-[2-(4-fluorophényl)pyrazolo[1,5-*a*]pyridin-5-yl]phényl}but-3-yn-2-ol est élué en deuxième position (temps de rétention = 44 min) lors de la séparation effectuée dans l'exemple 11. Après concentration sous pression réduite, on obtient 0,083 g de produit attendu sous forme d'une poudre blanche.
Point de fusion (°C) : 126-128.
LC-MS : M+H = 357
RMN-¹H (DMSO) δ (ppm) : 1,87 (s, 3H) ; 5,45 (m, 1H) ; 5,95 (d, 1H) ; 7,12 (s, 1H) ; 7,25 (dd, 1H) ; 7,32 (t, 2H) ; 7,53 (m, 2H) ; 7,75 (m, 1H) ; 7,86 (s, 1H) ; 7,99 (m, 1H) ; 8,08 (m, 2H) ; 8,78 (d, 1H).
Pouvoir rotatoire: α_{D} = +18 ° (c=0,244 g/dL, MeOH, 589 nm)
Pureté énantiomérique : 99,5% (sur colonne CHIRALPAK AD-H 5 µm (250*4,6 mm) avec pour éluant un mélange d'hexane/éthanol/méthanol 70/15/15 (1mL/min), temps de rétention de 21,10 min)

Les tableaux qui suivent illustrent les structures chimiques de formule générale (I) (tableau 1) et les caractéristiques physicochimiques (tableau 2) de quelques exemples de composés selon l'invention.

Dans ces tableaux :
- la colonne « PF » renseigne les points de fusion des produits en degrés Celsius (°C).
- la colonne « Position » renseigne sur la position de substitution du groupe 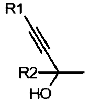 sur le noyau phényle (2, 3 ou 4) ;

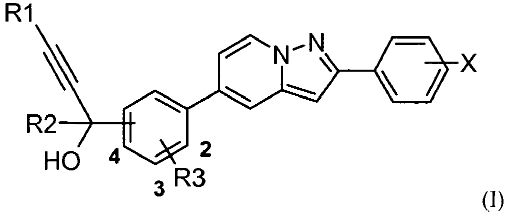

**Tableau 1**

| **N°** | **R1** | **R2** | **R3** | **Position** | **x** | *Chiralité* |
|---|---|---|---|---|---|---|
| **1** | H | H | H | 3 | 4-Cl | (racémique) |
| **2** | H | H | H | 3 | 4-F | (racémique) |
| **3** | Me | H | H | 3 | 4-F | (racémique) |
| **4** | H | Me | H | 3 | 4-F | (racémique) |
| **5** | H | H | H | 2 | 4-F | (racémique) |
| **6** | H | H | 2,4-diF | 3 | 4-F | (racémique) |
| **7** | H | H | H | 3 | 2-F | (racémique) |
| **8** | H | H | H | 3 | 4-Me | (racémique) |
| **9** | H | H | H | 3 | 4-F | Lévogyre (DMSO) |
| **10** | H | H | H | 3 | 4-F | Dextrogyre (DMSO) |
| **11** | Me | H | H | 3 | 4-F | Lévogyre (MeOH) |
| **12** | Me | H | H | 3 | 4-F | Dextrogyre (MeOH) |

| | | | | | | |
|---|---|---|---|---|---|---|
| (le solvant utilisé pour la détermination du pouvoir rotatoire est précisé entre parenthèses) | | | | | | |

**Tableau 2**

| **N°** | **PF** | **RMN** / **[M+H]** |
|---|---|---|
| **1** | 195-197 | RMN-¹H (DMSO) δ (ppm) : 3,55 (s, 1H) ; 5,50 (m, 1H) ; 6,15 (d, 1H) ; 7,20 (s, 1H) ; 7,30 (d, 1H) ; 7,60 (m, 4H) ; 7,80 (m, 1H) ; 7,90 (s, 1H) ; 8,00 (m, 3H) ; 8,80 (d, 1H). M+H= 359 |
| **2** | 192-194 | RMN-¹H (DMSO) δ (ppm) : 3,55 (s, 1H) ; 5,50 (m, 1H) ; 6,15 (d, 1H) ; 7,10 (s, 1H) ; 7,20 (d, 1H) ; 7,35 (m, 2H) ; 7,55 (q, 2H) ; 7,75 (q, 1H) ; 7,90 (s, 1H) ; 8,00 (s, 1H) ; 8,10 (m, 2H) ; 8,80 (d, 1H). M+H= 343 |
| **3** | 164-166 | RMN-¹H (DMSO) δ (ppm) : 1,87 (s, 3H) ; 5,45 (m, 1H) ; 5,95 (d, 1H) ; 7,12 (s, 1H) ; 7,25 (dd, 1H) ; 7,32 (t, 2H) ; 7,53 (m, 2H) ; 7,75 (m, 1H) ; 7,86 (s, 1H) ; 7,99 (m, 1H) ; 8,08 (m, 2H) ; 8,78 (d, 1H). M+H = 357 |
| **4** | 215-217 | RMN-¹H (DMSO) δ (ppm) : 1,75 (s, 3H) ; 3,58 (s, 1H) ; 6,20 (s, 1H) ; 7,12 (s, 1H) ; 7,25 (m, 1H) ; 7,32 (t, 2H) ; 7,53 (m, 1H) ; 7,65 (m, 1H) ; 7,75 (m, 1H) ; 7,98 (m, 2H) ; 8,08 (m, 2H) ; 8,80 (d, 1H).M+H = 357 |
| **5** | 104-106 | RMN-¹H (DMSO) δ (ppm) : 3,50 (s, 1H) ; 5,35 (m, 1H) ; 6,12 (m, 1H) ; 6,95 (dd, 1H) ; 7,12 (s, 1H) ; de 7,30 à 7,40 (m, 3H) ; 7,46 (m, 1H) ; 7,52 (m, 1H) ; 7,69 (m, 1H) ; 7,83 (m, 1H) ; 8,07 (m, 2H) ; 8,78 (d, 1H). M+H = 343 |
| **6** | 151-153 | RMN-¹H (DMSO) δ (ppm) : 3,50 (s, 1H) ; 5,75 (s, 1H) ; 6,28 (d, 1H) ; 7,05 (m, 1H) ; 7,15 (s, 1H) ; de 7,25 à 7,38 (m, 3H) ; 7,70 (m, 1H) ; 7,85 (s, 1H) ; 8,05 (m, 2H) ; 8,78 (d, 1H). M+H = 379 |
| **7** | 157-159 | RMN-¹H (DMSO) δ (ppm) : 3,55 (d, 1H) ; 5,55 (m, 1H) ; 6,15 (m, 1H) ; 7,12 (m, 1H) ; 7,30 (m, 1H) ; de 7,32 à 7,45 (m, 2H) ; de 7,46 à 7,52 (m, 1H) ; 7,55 (m, 2H) ; 7,78 (m, 1H) ; 7,90 (s, 1H) ; 8,08 (s, 1H) ; 8,15 (m, 1H) ; 8,82 (d, 1 H). M+H = 343 |
| **8** | 183-185 | RMN-¹H (DMSO) δ (ppm) : 2,40 (s, 3H) ; 3,55 (d, 1H) ; 5,55 (m, 1H) ; 6,15 (m, 1H) ; 7,10 (s, 1H) ; 7,22 (m, 1H) ; 7,32 (m, 2H) ; 7,55 (m, 2H) ; 7,78 (m, 1H) ; de 7,90 à 7,95 (m, 3H) ; 7,95 (s, 1H) ; 8,80 (d, 1H). M+H= 339 |
| **9** | 147-149 | RMN-¹H (DMSO) δ (ppm) : 3,55 (s, 1H) ; 5,50 (m, 1H) ; 6,15 (d, 1H) ; 7,10 (s, 1H) ; 7,20 (d, 1H) ; 7,35 (m, 2H) ; 7,55 (q, 2H) ; 7,75 (q, 1H) ; 7,90 (s, 1H) ; 8,00 (s, 1H) ; 8,10 (m, 2H) ; 8,80 (d, 1H). M+H= 343 |
| **10** | 139-141 | RMN-¹H (DMSO) δ (ppm) : 3,55 (s, 1H) ; 5,50 (m, 1H) ; 6,15 (d, 1H) ; 7,10 (s, 1H) ; 7,20 (d, 1H) ; 7,35 (m, 2H) ; 7,55 (q, 2H) ; 7,75 (q, 1H) ; 7,90 (s, 1H) ; 8,00 (s, 1H) ; 8,10 (m, 2H) ; 8,80 (d, 1H).M+H= 343 |
| **11** | 122-124 | RMN-¹H (DMSO) δ (ppm) : 1,87 (s, 3H) ; 5,45 (m, 1H) ; 5,95 (d, 1H) ; 7,12 (s, 1H) ; 7,25 (dd, 1H) ; 7,32 (t, 2H) ; 7,53 (m, 2H) ; 7,75 (m, 1H) ; 7,86 (s, 1H) ; 7,99 (m, 1H) ; 8,08 (m, 2H) ; 8,78 (d, 1H). M+H = 357 |
| **12** | 126-128 | RMN-¹H (DMSO) δ (ppm) : 1,87 (s, 3H) ; 5,45 (m, 1H) ; 5,95 (d, 1H) ; 7,12 (s, 1H) ; 7,25 (dd, 1H) ; 7,32 (t, 2H) ; 7,53 (m, 2H) ; 7,75 (m, 1H) ; 7,86 (s, 1H) ; 7,99 (m, 1H) ; 8,08 (m, 2H) ; 8,78 (d, 1H). M+H = 357 |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture, les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25 µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.

Les meilleurs composés ont une CE50 comprise entre 0,1 nM et 10 µM.

Par exemple, le composé n°2 a montré une CE50 de 3 nM.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto-temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaques, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcéreuse; les maladies inflammatoires allergiques telle que l'asthme, les maladies auto-immunes comme le diabète de type 1, lupus, sclérodermie, syndrome de Guillain-Barré, maladie d'Addison et autres maladies inmuno-médiées; l'ostéoporose; les cancers.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés. Selon la pratique habituelle, le dosage approprié à chaque patient est déterminé par le médecin selon le mode d'administration, le poids et la réponse dudit patient.

## Revendications

1. Composés de formule (I) : 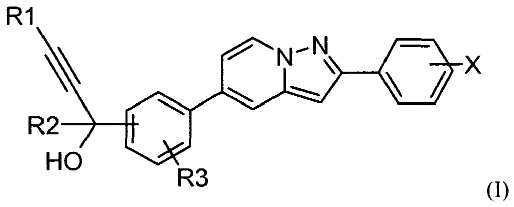 dans laquelle :
R₁ et R2 représentent, indépendamment l'un de l'autre, un atome d'hydrogène ou un groupe (C₁-C₆)alkyle,
R3 représente un ou plusieurs atomes d'hydrogène ou d'halogène,
X représente de 1 à 4 substituants identiques ou différents l'un de l'autre choisis parmi hydrogène, halogène ou (C₁-C₆)alkyle,
à l'état de base ou de sel d'addition à un acide.

2. Composés de formule (I) selon la revendication 1, **caractérisés en ce que** X représente un atome d'halogène ou un groupe (C₁-C₆)alkyle;
R1 et R2 représentent un atome d'hydrogène ou un groupe (C₁-C₆)alkyle ;
R3 représente ou plusieurs atomes d'hydrogène ou d'halogène, à l'état de base ou de sel d'addition à un acide.

3. Composés de formule (I) selon la revendication 1, **caractérisé en ce que** X représente un chlore, un fluor ou un groupe méthyle;
R1 et R2 représentent un atome d'hydrogène ou un groupe méthyle ;
R3 représente un atome d'hydrogène ou un groupe difluoro, à l'état de base ou de sel d'addition à un acide.

4. Composés
1-{3-[2-(4-Chlorophényl)pyrazolo[1,5-*a*]pyridin-5-yl]phényl}prop-2-yn-1-ol
1-{3-[2-(4-Fluorophényl)pyrazolo[1,5-*a*]pyridin-5-yl]phényl}prop-2-yn-1-ol
1-{3-[2-(4-Fluorophényl)pyrazolo[1,5-*a*]pyridin-5-yl]phényl}but-2-yn-1-ol
2-{3-[2-(4-Fluorophényl)pyrazolo[1,5-*a*]pyridin-5-yl]phényl}but-3-yn-2-ol
1-{2-[2-(4-Fluoro-phényl)pyrazolo[1,5-a]pyridin-5-yl]phényl}prop-2-yn-1-ol
1-{2,6-Difluoro-3-[2-(4-fluorophényl)pyrazolo[1,5-*a*]pyridin-5-yl]phényl}prop-2-yn-1-ol
1-{3-[2-(2-Fluorophényl)pyrazolo[1,5-*a*]pyridin-5-yl]phényl}prop-2-yn-1-ol
1-[3-(2-p-Tolylpyrazolo[1,5-*a*]pyridin-5-yl)phényl]prop-2-yn-1-ol
(-)-1-{3-[2-(4-Fluorophényl)pyrazolo[1,5-*a*]pyridin-5-yl]phényl}prop-2-yn-1-ol
(+)-1-{3-[2-(4-fluorophényl)pyrazolo[1,5-*a*]pyridin-5-yl]phényl}prop-2-yn-1-ol
(-)-2-{3-[2-(4-Fluorophényl)pyrazolo[1,5-*a*]pyridin-5-yl]phényl}but-3-yn-2-ol
(+)-2-{3-[2-(4-Fluorophényl)pyrazolo[1,5-*a*]pyridin-5-yl]phényl}but-3-yn-2-ol

5. Médicament, **caractérisé en ce qu'**il comprend un composé de formule (I) selon l'une quelconque des revendications 1 à 4, ou un sel d'addition de ce composé à un acide pharmaceutiquement acceptable.

6. Composition pharmaceutique, **caractérisée en ce qu'**elle comprend un composé de formule (I) selon l'une quelconque des revendications 1 à 4, ou un sel phannaceutiquement acceptable, de ce composé, ainsi qu'au moins un excipient pharmaceutiquement acceptable.

7. Utilisation d'un composé de formule (I) selon l'une quelconque des revendications 1 à 4 pour la préparation d'un médicament destiné au traitement et à la prévention des maladies neurodégénératives.

8. Utilisation d'un composé de formule (I) selon l'une quelconque des revendications 1 à 4 pour la préparation d'un médicament destiné au traitement et à la prévention des traumatismes cérébraux et de l'épilepsie.

9. Utilisation d'un composé de formule (I) selon l'une quelconque des revendications 1 à 4 pour la préparation d'un médicament destiné au traitement et à la prévention des maladies psychiatriques.

10. Utilisation d'un composé de formule (I) selon l'une quelconque des revendications 1 à 4 pour la préparation d'un médicament destiné au traitement et à la prévention des maladies inflammatoires.

11. Utilisation d'un composé de formule (I) selon l'une quelconque des revendications 1 à 4 pour la préparation d'un médicament destiné au traitement et à la prévention de l'ostéoporose et les cancers.

12. Utilisation d'un composé de formule (I) selon l'une quelconque des revendications 1 à 4 pour la préparation d'un médicament destiné au traitement et à la prévention de la maladie de Parkinson, d'Alzheimer, des tauopathies, de la sclérose en plaque.

13. Utilisation d'un composé de formule (I) selon l'une quelconque des revendications 1 à 4 pour la préparation d'un médicament destiné au traitement et à la prévention de la schizophrénie, la dépression, la dépendance à une substance, les troubles du déficit de l'attention et de l'hyperactivité.

## Patentansprüche

1. Verbindungen der Formel (I): 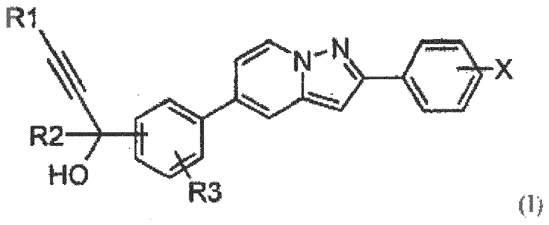 worin:
R1 und R2 unabhängig voneinander für ein Wasserstoffatom oder eine (C₁-C₆)-Alkylgruppe stehen,
R3 für ein oder mehrere Wasserstoff- oder Halogenatome steht,
X für 1 bis 4 gleiche oder voneinander verschiedene Substituenten, die aus Wasserstoff,
Halogen oder (C₁-C₆)-Alkyl ausgewählt sind, steht, in Basen- oder Säureadditionssalzform.

2. Verbindungen der Formel (I) nach Anspruch 1, **dadurch gekennzeichnet, dass**
X für ein Halogenatom oder eine (C₁-C₆)-Alkylgruppe steht;
R1 und R2 für ein Wasserstoffatom oder eine (C₁-C₆)-Alkylgruppe stehen;
R3 für ein oder mehrere Wasserstoff- oder Halogenatome steht,
in Basen- oder Säureadditionssalzform.

3. Verbindungen der Formel (I) nach Anspruch 1, **dadurch gekennzeichnet, dass**
X für ein Chlor, ein Fluor oder eine Methylgruppe steht;
R1 und R2 für ein Wasserstoffatom oder eine Methylgruppe stehen;
R3 für ein Wasserstoffatom oder eine Difluorgruppe steht,
in Basen- oder Säureadditionssalzform.

4. Verbindungen
1-{3-[2-(4-chlorphenyl)pyrazolo[1,5-a]pyridin-5-yl]phenyl}prop-2-in-1-ol
1-{3-[2-(4-Fluorphenyl)pyrazolo[1,5-*a*]pyridin-5-yl]phenyl}prop-2-in-1-ol
1-{3-[2-(4-Fluorphenyl)pyrazolo[1,5-a]pyridin-5-yl]phenyl}but-2-in-1-ol
2-{3-[2-(4-Fluorphenyl)pyrazolo[1,5-a]pyridin-5-yl]phenyl}but-3-in-2-ol
1-{2-[2-(4-Fluorphenyl)pyrazolo[1,5-a]pyridin-5-yl]phenyl}prop-2-in-1-ol
1-{2,6-Difluor-3-[2-(4-fluorphenyl)pyrazolo[1,5-a]pyridin-5-yl]phenyl}prop-2-in-1-ol
1-{3-[2-(2-Fluorphenyl)pyrazolo[1,5-a]pyridin-5-yl]phenyl}prop-2-in-1-ol
1-[3-(2-p-Tolylpyrazolo[1,5-*a*]pyridin-5-yl)-phenyl]prop-2-in-1-ol
(-)-1-{3-[2-(4-Fluorphenyl)pyrazolo[1,5-*a*]pyridin-5-yl]phenyl}prop-2-in-1-ol
(+)-1-{3-[2-(4-Fluorphenyl)pyrazolo[1,5-*a*]pyridin-5-yl]phenyl}prop-2-in-1-ol
(-)-2-{3-[2-(4-Fluorphenyl)pyrazolo[1,5-*a*]pyridin-5-yl]phenyl}but-3-in-2-ol
(+)-2-{3-[2-(4-Fluorphenyl)pyrazolo[1,5-*a*]pyridin-5-yl]phenyl}but-3-in-2-ol.

5. Medikament, **dadurch gekennzeichnet, dass** es eine Verbindung der Formel (I) nach einem der Ansprüche 1 bis 4 oder ein Additionssalz dieser Verbindung mit einer pharmazeutisch unbedenklichen Säure umfasst.

6. Pharmazeutische Zusammensetzung, **dadurch gekennzeichnet, dass** sie eine Verbindung der Formel (I) nach einem der Ansprüche 1 bis 4 oder ein pharmazeutisch unbedenkliches Salz dieser Verbindung sowie mindestens einen pharmazeutisch unbedenklichen Hilfsstoff umfasst.

7. Verwendung einer Verbindung der Formel (I) nach einem der Ansprüche 1 bis 4 zur Herstellung eines Medikaments zur Behandlung oder Prävention von neurodegenerativen Erkrankungen.

8. Verwendung einer Verbindung der Formel (I) nach einem der Ansprüche 1 bis 4 zur Herstellung eines Medikaments zur Behandlung oder Prävention von Hirntraumen und Epilepsie.

9. Verwendung einer Verbindung der Formel (I) nach einem der Ansprüche 1 bis 4 zur Herstellung eines Medikaments zur Behandlung oder Prävention von psychiatrischen Erkrankungen.

10. Verwendung einer Verbindung der Formel (I) nach einem der Ansprüche 1 bis 4 zur Herstellung eines Medikaments zur Behandlung oder Prävention von entzündlichen Erkrankungen.

11. Verwendung einer Verbindung der Formel (I) nach einem der Ansprüche 1 bis 4 zur Herstellung eines Medikaments zur Behandlung oder Prävention von Osteoporose und Krebserkrankungen.

12. Verwendung einer Verbindung der Formel (I) nach einem der Ansprüche 1 bis 4 zur Herstellung eines Medikaments zur Behandlung oder Prävention von Parkinson-Krankheit, Alzheimer-Krankheit, Tauopathien und multipler Sklerose.

13. Verwendung einer Verbindung der Formel (I) nach einem der Ansprüche 1 bis 4 zur Herstellung eines Medikaments zur Behandlung oder Prävention von Schizophrenie, Depression, Substanzabhängigkeit und Aufmerksamkeitsdefizit-Hyperaktivitätssyndrom.

## Claims

1. Compounds of formula (I) : 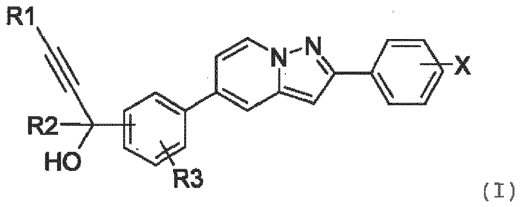 in which:
R1 and R2 represent, independently of one another, a hydrogen atom or a (C₁-C₆)alkyl group,
R3 represents one or more hydrogen or halogen atoms,
X represents from 1 to 4 substituents, identical to or different from one another, chosen from hydrogen, halogen or (C₁-C₆)alkyl,
in the form of the base or of an addition salt with an acid.

2. Compounds of formula (I) according to Claim 1, **characterized in that**
X represents a halogen atom or a (C₁-C₆)alkyl group;
R1 and R2 represent a hydrogen atom or a (C₁-C₆)alkyl group;
R3 represents one or more hydrogen or halogen atoms,
in the form of the base or of an addition salt with an acid.

3. Compounds of formula (I) according to Claim 1, **characterized in that**
X represents a chlorine, a fluorine or a methyl group;
R1 and R2 represent a hydrogen atom or a methyl group;
R3 represents a hydrogen atom or a difluoro group,
in the form of the base or of an addition salt with an acid.

4. Compounds
1-{3-[2-(4-Chlorophenyl)pyrazolo[1,5-a]pyridin-5-yl]phenyl}prop-2-yn-l-ol
1-{3-[2-(4-Fluorophenyl)pyrazolo[1,5-a]pyridin-5-yl]phenyl}prop-2-yn-l-ol
1-{3-[2-(4-Fluorophenyl)pyrazolo[1,5-*a*]pyridin-5-yl]phenyl}but-2-yn-l-ol
2-{3-[2-(4-Fluorophenyl)pyrazolo[1,5-*a*]pyridin-5-yl]phenyl}but-3-yn-2-ol
1-{2-[2-(4-Fluorophenyl)pyrazolo[1,5-a]pyridin-5-yl]phenyl}prop-2-yn-1-ol
1-{2,6-Difluoro-3-[2-(4-fluorophenyl)pyrazolo[1,5-*a*]pyridin-5-yl]phenyl}prop-2-yn-l-ol
1-{3-[2-(2-Fluorophenyl)pyrazolo[1,5-*a*]pyridin-5-yl]phenyl}prop-2-yn-l-ol
1-[3-(2-{p-Tolyl}pyrazolo[1,5-*a*]pyridin-5-yl)phenyl]prop-2-yn-1-ol
(-)-1-{3-[2-(4-Fluorophenyl)pyrazolo[1,5-*a*]pyridin-5-yl]phenyl}prop-2-yn-l-ol
(+)-1-{3-[2-(4-Fluorophenyl)pyrazolo[1,5-*a*]pyridin-5-yl]phenyl}prop-2-yn-l-ol
(-)-2-{3-[2-(4-Fluorophenyl)pyrazolo[1,5-*a*]pyridin-5-yl]phenyl}but-3-yn-2-ol
(+)-2-{3-[2-(4-Fluorophenyl)pyrazolo[1,5-*a*]pyridin-5-yl]phenyl}but-3-yn-2-ol

5. Medicament, **characterized in that** it comprises a compound of formula (I) according to any one of Claims 1 to 4 or an addition salt of this compound with a pharmaceutically acceptable acid.

6. Pharmaceutical composition, **characterized in that** it comprises a compound of formula (I) according to any one of Claims 1 to 4, or a pharmaceutically acceptable salt of this compound, and at least one pharmaceutically acceptable excipient.

7. Use of a compound of formula (I) according to any one of Claims 1 to 4 in the preparation of a medicament intended for the treatment or prevention of neurodegenerative diseases.

8. Use of a compound of formula (I) according to any one of Claims 1 to 4 in the preparation of a medicament intended for the treatment or prevention of cerebral traumas and epilepsy.

9. Use of a compound of formula (I) according to any one of Claims 1 to 4 in the preparation of a medicament intended for the treatment or prevention of psychiatric diseases.

10. Use of a compound of formula (I) according to any one of Claims 1 to 4 in the preparation of a medicament intended for the treatment or prevention of inflammatory diseases.

11. Use of a compound of formula (I) according to any one of Claims 1 to 4 in the preparation of a medicament intended for the treatment or prevention of osteoporosis and cancers.

12. Use of a compound of formula (I) according to any one of Claims 1 to 4 in the preparation of a medicament intended for the treatment or prevention of Parkinson's disease, Alzheimer's disease, tauopathies or multiple sclerosis.

13. Use of a compound of formula (I) according to any one of Claims 1 to 4 in the preparation of a medicament intended for the treatment or prevention of schizophrenia, depression, substance dependence or attention deficit hyperactivity disorders
